Europäisches Patentamt

European Patent Office

Office européen des brevets

(11) Numéro de publication : **0 356 315 B1**

# (12) FASCICULE DE BREVET EUROPEEN

(45) Date de publication du fascicule du brevet :
**08.07.92 Bulletin 92/28**

(51) Int. Cl.$^5$ : **C07C 57/04,** C07C 51/48,
C07C 51/377, C07C 69/54,
C07C 67/08

(21) Numéro de dépôt : **89402286.2**

(22) Date de dépôt : **11.08.89**

(54) **Procédé pour la fabrication de méthacrylate de méthyle à partir d'acide isobutyrique.**

(30) Priorité : **16.08.88 FR 8810911**

(43) Date de publication de la demande :
**28.02.90 Bulletin 90/09**

(45) Mention de la délivrance du brevet :
**08.07.92 Bulletin 92/28**

(84) Etats contractants désignés :
**AT BE CH DE ES FR GB GR IT LI LU NL SE**

(56) Documents cités :
**FR-A- 1 393 173
FR-A- 2 224 429
US-A- 2 541 486**

(73) Titulaire : **ELF ATOCHEM S.A.
4 & 8, Cours Michelet La Défense 10
F-92800 Puteaux (FR)**

(72) Inventeur : **Samuel, Yves
46, Rue Léopold Durand
F-57500 Saint Avold (FR)**
Inventeur : **Cauvy, Daniel
43, Rue Saint Hubert
Bambiderstroff F-57220 Boulay (FR)**

(74) Mandataire : **Rochet, Michel et al
ELF ATOCHEM S.A. Département Propriété
Industrielle 4-8, Cours Michelet La Défense 10
- Cedex 42
F-92091 Paris-La-Défense (FR)**

EP 0 356 315 B1

Jouve, 18, rue Saint-Denis, 75001 PARIS

## Description

La présente invention porte sur un procédé perfectionné pour la fabrication de méthacrylate de méthyle à partir d'acide isobutyrique.

Il est connu actuellement de préparer du méthacrylate de méthyle à partir d'acide isobutyrique par un procédé en deux étapes :

    – A la première étape, on effectue l'oxydéshydrogénation de l'acide isobutyrique, en phase vapeur, entre 300°C et 450°C, sur un catalyseur qui peut, soit être dérivé des phosphates de fer, comme cela est décrit, par exemple, dans le brevet américain n° 4 298 755, soit être dérivé de l'acide phosphomolybdique, comme cela est décrit, par exemple, dans le brevet européen n° 0 194 541.

La conversion de l'acide isobutyrique est généralement comprise entre 85% et 98%. De l'eau de dilution est généralement nécessaire pour maintenir l'activité du catalyseur, et de l'eau se forme durant la réaction. Le produit sortant du réacteur d'oxydéshydrogénation, après condensation, est principalement composé d'acide méthacrylique, d'acide isobutyrique non converti, d'eau en proportion plus ou moins importante (25 à 85%), ainsi que d'acétone et d'acide acétique en proportions moindres. Une façon connue de séparer l'eau des acides organiques consiste à effectuer une extraction liquide-liquide. A cet effet, de nombreux solvants d'extraction sont décrits dans la littérature ; on peut citer notamment les esters, les cétones, les solvants aromatiques et leurs mélanges.

Il est cependant nécessaire de procéder encore à un certain nombre de séparations pour obtenir un acide méthacrylique destiné à être soumis à la deuxième étape; ainsi, il faut éliminer l'acétone et il est souhaitable d'éliminer également l'acide acétique et l'acide isobutyrique.

    – A la deuxième étape, on effectue l'estérification de l'acide méthacrylique en méthacrylate de méthyle par du méthanol, suivant une réaction équilibrée, en phase liquide, entre 70°C et 130°C, en présence d'un catalyseur acide, qui peut être constitué, par exemple, par de l'acide sulfurique, de l'acide paratoluène sulfonique, de l'acide méthane sulfonique ou, de façon préférée, des résines cationiques fortes. A la sortie du réacteur, on récupère du méthacrylate de méthyle, de l'acide méthacrylique, de l'eau et du méthanol et, éventuellement, selon le degré de purification de l'acide méthacrylique introduit, des acides et esters acétiques et isobutyriques. Une colonne est généralement placée à la sortie du réacteur, permettant de soutirer l'acide ou les acides non convertis pour les recycler au réacteur d'estérification.

La purification du méthacrylate de méthyle est ensuite réalisée selon des procédés connus, comme des distillations, éventuellement, une extraction liquide-liquide, etc.

Le procédé de fabrication du méthacrylate de méthyle, tel qu'il vient d'être présenté, possède l'inconvénient majeur qu'à la première étape, l'acide isobutyrique est converti de manière incomplète en acide méthacrylique, si bien qu'en fin de compte, le méthacrylate de méthyle renferme, comme impureté, de l'isobutyrate de méthyle, en une quantité qui est supérieure à la limite autorisée pour qu'un méthacrylate de méthyle soit considéré comme un produit commercial de grande pureté. Un tel produit doit en effet contenir moins de 100 parties par million de substances non-polymérisables, autrement dit présenter une teneur en isobutyrate de méthyle inférieure à ce seuil de 100 parties par million.

Pour obtenir cette teneur très faible en isobutyrate de méthyle, deux possibilités s'offrent :

    – On peut imaginer de séparer, en fin de première étape, l'acide méthacrylique de l'acide isobutyrique, jusqu'à obtention d'un acide méthacrylique de pureté suffisante, c'est-à-dire contenant moins de 100 parties par million d'acide isobutyrique, l'acide isobutyrique obtenu en tête de colonne pouvant alors être recyclé au réacteur d'oxydéshydrogénation pour être combiné avec l'acide isobutyrique d'origine.

Cependant, la technologie de cette distillation est difficile à mettre en oeuvre. Les mesures d'équilibre liquide-vapeur montrent que, du fait d'un comportement thermodynamique non idéal, la séparation nécessite un très grand nombre de plateaux théoriques. Simultanément, du fait de la sensibilité à la polymérisation de l'acide méthacrylique et de son point d'ébullition élevé, il est nécessaire de conduire cette distillation sous un vide poussé et à l'aide d'un équipement effectuant la séparation avec une très faible perte de charge.

    – On peut aussi imaginer de ne pas modifier la première étape, auquel cas on introduit, pour effectuer l'estérification de la seconde étape, un mélange d'acide méthacrylique et d'acide isobutyrique non converti. Ces acides sont convertis en esters en proportion équivalente, et il convient alors de séparer l'isobutyrate de méthyle du méthacrylate de méthyle jusqu'à obtention de la pureté désirée.

Cette séparation offre une difficulté technologique moindre que la séparation, évoquée ci-dessus, de l'acide isobutyrique. En revanche, il est sans intérêt au plan industriel de disposer de l'isobutyrate de méthyle que l'on récupèrerait en tête de colonne. Son brûlage conduit à un gaspillage économique inacceptable, et, si on le recycle au réacteur d'oxydéshydrogénation, l'isobutyrate non converti et le méthacrylate de méthyle obtenu créeront une perturbation importante dans la première étape.

En conclusion, aucune des deux possibilités qui viennent à l'esprit pour améliorer le procédé connu en vue de l'obtention d'un méthacrylate de méthyle de grande pureté, ne semble satisfaisante.

Par la demande de brevet français n° 2 224 429, on connaît un procédé de récupération d'acide méthacrylique et de méthanol dans lequel un effluent d'estérification (méthacrylate de méthyle, méthanol, eau) et un effluent d'oxydéshydrogénation (acide méthacrylique, eau, acide acétique) s'extraient réciproquement. Par le brevet américain n° 2 541 486, on connaît un procédé pour éliminer l'eau de liquides aqueux contenant un acide aliphatique.

La Société Déposante a donc recherché une solution acceptable au problème posé, ce qui a conduit à la présente invention.

Celle-ci a en effet pour objet un procédé de fabrication de méthacrylate de méthyle à partir d'acide isobutyrique, suivant lequel :

– on soumet l'acide isobutyrique de départ à une oxydéshydrogénation catalytique en phase vapeur, conduisant à un mélange d'eau, d'acide méthacrylique, et impuretés, dont l'acide isobutyrique non converti ;

– on condense ledit mélange et on procède à une extraction liquide-liquide, afin de séparer l'eau des acides organiques obtenus ;

– on procède à une estérification desdits acides par le méthanol, conduisant à un méthacrylate de méthyle brut, dont l'isobutyrate de méthyle constitue une impureté,

caractérisé par le fait que :

– on réalise l'extraction liquide-liquide par une fraction du méthacrylate de méthyle brut obtenu par la réaction d'estérification ;

– on envoie la phase organique résultant de cette extraction à un étage de séparation, situé immédiatement en aval de l'étage d'estérification, permettant de séparer, d'une part, le méthacrylate de méthyle brut constitué par la fraction de méthacrylate de méthyle produit par l'estérification et celle utilisée comme solvant d'extraction, et, d'autre part, les acides organiques constitués par la fraction des acides organiques obtenus en fin d'oxydéshydrogénation et celle des acides non estérifiés qui se trouvent ainsi recyclés ; et

– on procède à une purification de la fraction de méthacrylate de méthyle brut qui n'a pas été utilisée comme solvant d'extraction, afin de séparer, d'une part, du méthacrylate de méthyle pur, et, d'autre part, de l'isobutyrate de méthyle que l'on recycle à l'étage d'oxydéshydrogénation.

Un tel procédé présente de grandes différences d'avec celui de la demande de brevet français n° 2 224 429, suivant lequel le méthacrylate de méthyle contient des quantités significatives de méthanol et où de l'eau est ajoutée en milieu de colonne d'extraction. Le brevet américain n° 2 541 486 ne décrit pas non plus l'alimentation d'un extracteur par acide acrylique-acide isobutyrique-eau, et par méthacrylate de méthyle-isobutyrate de méthyle ; les étages de ce procédé (estérification, distillation azéotropique, colonne d'alcool) ont des fonctions différentes des étages correspondants du procédé selon la présente invention.

Conformément à des modes de réalisation particuliers du procédé selon l'invention :

– on débarrasse la phase organique issue de l'étage d'extraction liquide-liquide, des impuretés que constituent l'acétone et l'acétate de méthyle, avant d'adresser ladite phase à l'étage de séparation méthacrylate de méthyle brut/acides organiques ;

– on débarrasse de son eau résiduelle l'effluent de méthacrylate de méthyle brut, avant de renvoyer ledit effluent à l'étage d'extraction et à l'étage de séparation méthacrylate de méthyle brut/isobutyrate de méthyle ; par ailleurs, on peut prévoir de recycler, à titre de reflux, une fraction de l'effluent de méthacrylate de méthyle brut, débarrassé de l'eau résiduelle, à l'étage de séparation méthacrylate de méthyle brut/acides organiques ;

– on effectue une purification supplémentaire du méthacrylate de méthyle obtenu à l'étage de séparation méthacrylate de méthyle pur/isobutyrate de méthyle, les produits lourds séparés étant, le cas échéant, recyclés à l'étage de séparation méthacrylate de méthyle brut/acides organiques ;

– on recycle à l'étage d'oxydéshydrogénation la phase aqueuse soutirée à l'étape d'extraction, après avoir soumis ladite phase à un strippage permettant de séparer une phase organique, que l'on recycle à l'étage d'estérification, et après avoir soustrait de la phase aqueuse purifiée par le strippage, une purge sensiblement égale à l'eau formée par les réactions d'oxydéshydrogénation et d'estérification ; par ailleurs, on peut recycler à l'étage de strippage, l'eau résiduelle que l'on a pu extraire de l'effluent de méthacrylate de méthyle brut.

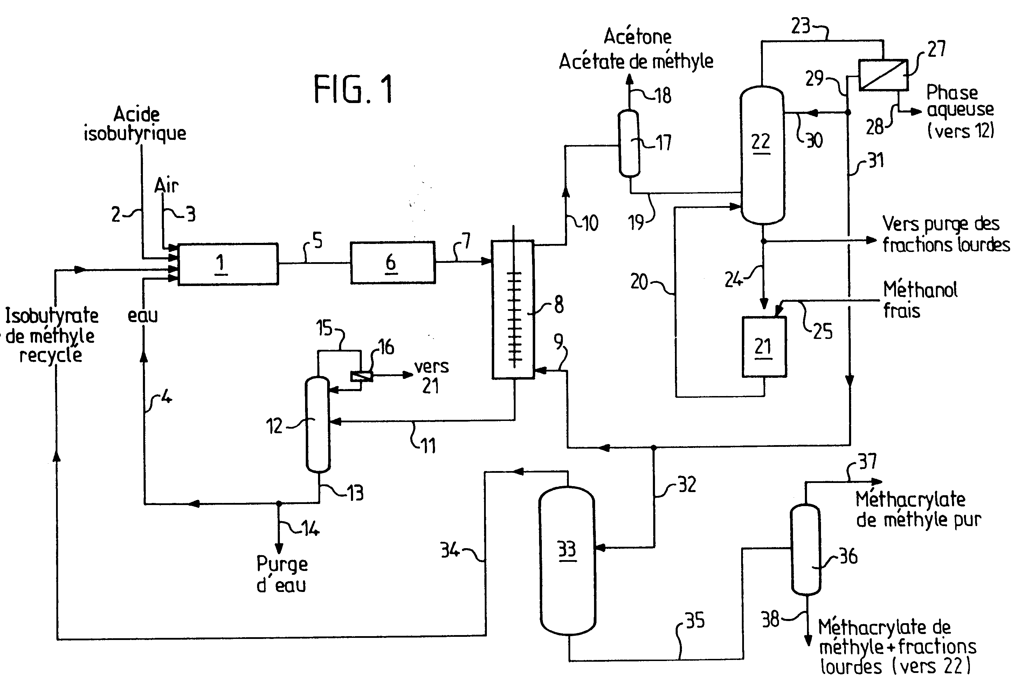

L'invention sera encore illustrée par la description qui suit d'un mode de réalisation préféré, faite en référence à la figure 1 du dessin annexé ; cette figure représente le diagramme de fonctionnement du procédé conforme à ce mode de réalisation préféré.

A l'étage 1 de ce diagramme, on effectue, de façon classique, l'oxydéshydrogénation catalytique de l'acide isobutyrique en phase vapeur ; l'étage 1 est alimenté en acide isobutyrique par la ligne 2, en air, par la ligne 3, et en eau, par la ligne 4, cette dernière constituant une ligne de recyclage comme cela sera décrit ci-après. De l'eau de dilution est en effet nécessaire pour maintenir l'activité du catalyseur, lequel est choisi notamment parmi les substances indiquées ci-dessus.

L'effluent sortant de cet étage 1, suivant la ligne 5, est un effluent gazeux formé par le mélange de l'eau de dilution, à laquelle s'ajoute l'eau formée durant la réaction, et de matières organiques principalement constituées par l'acide méthacrylique qui est le produit recherché, et, dans des proportions moindres, par l'acide isobutyrique non converti, et, en tant que sous-produits, de l'acétone, de l'acétate de méthyle et de l'acide acétique.

Cet effluent 5 est condensé à l'étage 6, puis le condensat 7 est adressé à l'étage 8 d'extraction liquide-liquide. Le solvant, entrant suivant la ligne d'extraction 9 à la partie inférieure de la colonne d'extraction 8, est constitué par une fraction recyclée de méthacrylate de méthyle brut, comme cela sera décrit ci-après. A la tête de la colonne d'extraction 8, on obtient une phase organique 10, et on soutire, à la partie inférieure de cette colonne 8, une phase aqueuse 11.

Cette dernière est adressée à mi-hauteur d'une colonne de strippage 12, de la partie inférieure de laquelle est soutirée une phase aqueuse circulant dans la ligne 13. Une purge d'eau 14, en particulier des eaux des réactions d'oxydéshydrogénation et d'estérification, est disposée sur cette ligne 13 ; cette eau, qui peut subir un traitement préalable, peut être rejetée en milieu naturel. On obtient alors l'effluent aqueux de la ligne 4, recyclé à l'étage d'oxydéshydrogénation 1. En tête de la colonne de strippage 12, sort une phase organique 15, laquelle, après condensation en 16, est recyclée vers l'étage d'estérification qui sera décrit ci-après.

Quant à l'effluent 10 sortant en tête de la colonne d'extraction liquide-liquide 8, il est adressé à mi-hauteur d'une colonne de distillation 17 en tête de laquelle est extrait, ce qui est symbolisé en 18, les impuretés que constituent l'acétone et l'acétate de méthyle. L'acide acétique présent dans le système est, soit oxydé dans le réacteur d'oxydéshydrogénation, soit transformé par la suite, à l'étage d'estérification décrit ci-après, en acétate de méthyle, et il finit pas être extrait en 18.

L'effluent 19, sortant de la partie inférieure de cette colonne 17, est adressé à une colonne de distillation 22, laquelle reçoit également, en un point inférieur, un courant 20 sortant de l'étage d'estérification 21. A titre de variante, l'effluent 19 reçoit le courant 20, le courant résultant étant adressé à la colonne 22. Le méthacrylate de méthyle brut est séparé, à la partie supérieure de la colonne 22, suivant la ligne 23, et à la partie inférieure de ladite colonne 22, sont soutirés, suivant la ligne 24, les acides organiques (acide méthacrylique, et dans une moindre mesure, acide isobutyrique) qui sont adressés à l'étage d'estérification 21. L'alimentation en méthanol frais est schématisée par la ligne 25. Toutefois, le courant 24 adressé à l'étage d'estérification 21, subit, avant de pénétrer dans ledit étage 21, une purge destinée à éliminer les substances lourdes, ce qui est symbolisé en 26.

L'effluent de la ligne 23 est condensé à l'étage 27, à partir duquel on sépare, d'une part, suivant la ligne 28, une phase aqueuse, avantageusement recyclée vers la colonne de strippage 12, par une ligne non représentée pour simplifier le dessin, et, d'autre part, suivant la ligne 29, du méthacrylate de méthyle brut. Ce dernier est, en partie recyclé à titre de reflux, suivant la ligne 30, à la tête de la colonne 22 précitée, et en partie adressé, par la ligne 31, d'une part, suivant la ligne 9, à la colonne d'extraction liquide-liquide 8, et, d'autre part, suivant la ligne 32, à un étage 33 de séparation méthacrylate de méthyle pur/isobutyrate de méthyle. Ainsi, l'isobutyrate de méthyle est prélevé à la partie supérieure de la colonne de distillation 33 pour être, suivant la conduite 34, recyclé à l'étage d'oxydéshydrogénation 1, et le méthacrylate de méthyle purifié est soutiré à la partie inférieure de la colonne 33, suivant la conduite 35, pour être adressé à mi-hauteur d'une seconde colonne 36, en vue d'une purification supplémentaire, permettant d'obtenir, en tête, suivant la ligne 37, le méthacrylate de méthyle très pur, et en pied de colonne, suivant la ligne 38, du méthacrylate de méthyle en combinaison avec des fractions lourdes, lequel est avantageusement recyclé vers la colonne de distillation 22, suivant une ligne non représentée pour simplifier le dessin.

Il est bien entendu que le mode de réalisation ci-dessus décrit a été donné à titre indicatif et que des modifications peuvent être apportées sans que l'on s'écarte pour autant du cadre de l'invention.

## Revendications

1. Procédé de fabrication de méthacrylate de méthyle à partir d'acide isobutyrique, suivant lequel :
   – on soumet l'acide isobutyrique de départ à une oxydéshydrogénation catalytique en phase vapeur (en 1), conduisant à un mélange (5) d'eau, d'acide méthacrylique et d'impuretés, dont l'acide isobutyrique non converti ;
   – on condense ledit mélange (en 6) et on procède à une extraction liquide-liquide (en 8), afin de séparer l'eau (en 11) des acides organiques obtenus (10) ;
   – on procède à une estérification (en 21), desdits acides par le méthanol, conduisant à un méthacrylate de méthyle brut, dont l'isobutyrate de méthyle constitue une impureté,
   caractérisé par le fait que :
   – on réalise l'extraction liquide-liquide (en 8) par une fraction (9) du méthacrylate de méthyle brut obtenu par la réaction d'estérification (en 21) ;
   – on envoie la phase organique (10) résultant de cette extraction à un étage de séparation (22), situé immédiatement en aval de l'étage d'estérification (21), permettant de séparer, d'une part, le méthacrylate de méthyle brut (23) constitué par la fraction de méthacrylate de méthyle produit par l'estérification et celle utilisée comme solvant d'extraction, et, d'autre part, les acides organiques (24) constitués par la fraction des acides organiques obtenus en fin d'oxydéshydrogénation et celle des acides non estérifiés qui se trouvent ainsi recyclés ; et

– on procède à une purification (en 33) de la fraction de méthacrylate de méthyle brut (32) qui n'a pas été utilisée comme solvant d'extraction, afin de séparer, d'une part, du méthacrylate de méthyle pur (en 35), et, d'autre part, de l'isobutyrate de méthyle (34) que l'on recycle à l'étage d'oxydéshydrogénation (1).

2. Procédé selon la revendication 1, caractérisé par le fait que l'on débarrasse (en 17) la phase organique (10) issue de l'étage d'extraction liquide-liquide (8), des impuretés que constituent l'acétone et l'acétate de méthyle, avant d'adresser (en 19) ladite phase à l'étage de séparation (22) méthacrylate de méthyle brut/acides organiques.

3. Procédé selon l'une des revendications 1 et 2, caractérisé par le fait que l'on débarrasse de son eau résiduelle (en 27) l'effluent de méthacrylate de méthyle brut (23), avant de renvoyer ledit effluent à l'étage d'extraction (8) et à l'étage de séparation (33) méthacrylate de méthyle brut/isobutyrate de méthyle.

4. Procédé selon la revendication 3, caractérisé par le fait que l'on recycle (en 30), à titre de reflux, une fraction de l'effluent de méthacrylate de méthyle brut, débarrassé (en 27) de l'eau résiduelle, à l'étage de séparation (22) méthacrylate de méthyle brut/acides organiques.

5. Procédé selon l'une des revendications 1 à 4, caractérisé par le fait que l'on effectue une purification supplémentaire (en 36) du méthacrylate de méthyle obtenu à l'étage de séparation (33) du méthacrylate de méthyle pur et de l'isobutyrate de méthyle, les produits lourds séparés (en 38) étant, le cas échéant, recyclés à l'étage de séparation (22) méthacrylate de méthyle brut/acides organiques.

6. Procédé selon l'une des revendications 1 à 5, caractérisé par le fait que l'on recycle à l'étage d'oxydéshydrogénation (1) la phase aqueuse (11) soutirée à l'étape d'extraction (en 8), après avoir soumis ladite phase à un strippage (en 12) permettant de séparer une phase organique que l'on recycle à l'étage d'estérification (21), et après avoir soustrait de la phase aqueuse purifiée par le strippage, une purge (14) sensiblement égale à l'eau formée par les réactions d'oxydéshydrogénation (en 1) et d'estérification (en 21).

7. Procédé selon les revendications 3 et 6 prises simultanément, caractérisé par le fait que l'on recycle à l'étage de strippage (12), l'eau résiduelle (28) extraite (en 27) de l'effluent (23) de méthacrylate de méthyle brut.

8. Procédé selon l'une des revendications 2 à 7, caractérisé par le fait que le courant (20) sortant de l'étage d'estérification (19) est adressé à l'étage de séparation (22) en un point inférieur à celui où est adressé le courant (19) issu de l'étage d'extraction liquide-liquide (8), après avoir été débarrassé (en 27) des impuretés qu'il contenait.

9. Procédé selon l'une des revendications 2 à 7, caractérisé par le fait qu'avant d'être adressé à l'étage de séparation (22), le courant (20) sortant de l'étage d'estérification (19) est combiné au courant (19) issu de l'étage d'extraction liquide-liquide (8), après avoir été débarrassé (en 27) des impuretés qu'il contenait.

**Patentansprüche**

1. Verfahren zur Herstellung von Methylmethacyrlat aus Isobuttersäure, bei welchem man:

– die eingesetzte Isobuttersäure einer katalytischen Oxydehydrierung in der Dampfphase unterwirft (bei 1), welche zu einer Mischung (5) aus Wasser, Methacrylsäure und Verunreinigungen, darunter die nicht umgesetzte Isobuttersäure, führt,

– die genannte Mischung kondensiert (bei 6) und eine Flüssig-Flüssig-Extraktion vornimmt (bei 8), um das Wasser (bei 11) von den erhaltenen organischen Säuren (10) abzutrennen,

– eine Veresterung (bei 21) der genannten Säuren mit Methanol vornimmt, was zu einem Roh-Methylmethacrylat führt, worin das Isomethylbutyrat eine Verunreinigung bildet,

dadurch gekennzeichnet, daß man

– die Flüssig-Flüssig-Extraktion (bei 8) mit einer Fraktion (9) des durch die Veresterungsreaktion (bei 21) erhaltenen Roh-Methylmethacrylats vornimmt,

– die aus dieser Extraktion resultierende organische Phase (10) zu einer unmittelbar stromab der Veresterungsstufe (21) gelegenen Trennstufe (22) führt, die erlaubt, einerseits das Roh-Methylmethacrylat (23), das von der durch die Veresterung erzeugten Methylmethacrylat-Fraktion und der als Extraktionslösungsmittel verwendeten Fraktion gebildet wird, und anderseits die organischen Säuren (24), die von der Fraktion der am Ende der Oxydehydrierung erhaltenen organischen Säuren und der Fraktion der nicht veresterten und somit rückgeführten Säuren, gebildet werden, abzutrennen, und

– eine Reinigung (bei 33) der Roh-Methylmethacrylat-Fraktion (32), die nicht als Extraktionslösungsmittel verwendet worden ist, vornimmt, um einerseits reines Methylmethacrylat (bei 35) und anderseits das Isomethylbutyrat (34) abzutrennen, das man zur Oxydehydrierungsstufe (1) zurückführt.

2. Verfahren nach Anspruch 1, dadurch gekennzeichnet, daß man (bei 17) die von der Flüssig-Flüssig-Extraktionsstufe (8) austretende organische Phase von den von Aceton und Methylacetat gebildeten Verunreinigungen befreit, bevor die genannte Phase (bei 19) zur Roh-Methylmethacrylat/organische Säuren-Trennstufe (22) geschickt wird.

3. Verfahren nach Anspruch 1 oder 2, dadurch gekennzeichnet, daß man den Roh-Methylmethacrylat-Abfluß (23) von seinem Restwasser befreit (bei 27), bevor der genannte Abfluß zur Extraktionsstufe (8) und zur Roh-Methylmethacrylat/Isomethylbutyrat-Trennstufe (33) geschickt wird.

4. Verfahren nach Anspruch 3, dadurch gekennzeichnet, daß man (bei 30) eine Fraktion des (bei 27) vom Restwasser befreiten Roh-Methylmethacrylat-Abflusses als Rückfluß zur Roh-Methylmethacrylat/organische Säuren-Trennstufe (22) zurückführt.

5. Verfahren nach einem der Ansprüche 1 bis 4, dadurch gekennzeichnet, daß man eine zusätzliche Reinigung (bei 36) des in der Stufe (33) zum Abtrennen des reinen Methylmethacrylats und des Isomethylbutyrats erhaltenen Methylmethacrylats vornimmt, wobei die (bei 38) abgetrennten schweren Produkte gegebenenfalls zur Roh-Methylmethacrylat/organische Säuren-Trennstufe (22) rückgeführt werden.

6. Verfahren nach einem der Ansprüche 1 bis 5, dadurch gekennzeichnet, daß man die in der Extraktionsstufe (bei 8) abgezogene wäßrige Phase (11) zur Oxydehydrierungsstufe (1) zurückführt; nachdem die genannte Phase einem Stripping (bei 12) unterworfen wurde, das erlaubt, eine organische Phase abzutrennen, die man zur Veresterungsstufe (21) zurückführt, und nachdem aus der durch das Stripping gereinigten wäßrigen Phase eine Wasserentnahme (14) erfolgte, die im wesentlichen dem durch die Oxydehydrierungsreaktion (bei 1) und die Veresterungsreaktion (bei 21) gebildeten Wasser entspricht.

7. Verfahren nach den Ansprüchen 3 und 6, dadurch gekennzeichnet, daß man das (bei 27) abgezogene Restwasser (28) des Roh-Methylmethacrylat-Abflusses (23) zur Stripping-Stufe (12) zurückführt.

8. Verfahren nach einem der Ansprüche 2 bis 7, dadurch gekennzeichnet, daß der aus der Veresterungsstufe (19) austretende Strom (20) der Trennstufe (22) an einem Punkt zugeführt wird, der unter jenem liegt, an dem der aus der Flüssig-Flüssig-Extraktionsstufe (8) austretende Strom (19), nachdem er (bei 27) von den Verunreinigungen, die er enthielt, befreit worden ist, zugeführt wird.

9. Verfahren nach einem der Ansprüche 2 bis 7, dadurch gekennzeichnet, daß der aus der Veresterungsstufe (19) austretende Strom (20), bevor er zur Trennstufe (22) geführt wird, mit dem aus der Flüssig-Flüssig-Extraktionsstufe (8) austretenden Strom (19) vereinigt wird, nachdem letzterer (bei 27) von den Verunreinigungen, die er enthielt, befreit worden ist.

## Claims

1. Process for the manufacture of methyl methacrylate from isobutyric acid, according to which:
   - the starting isobutyric acid is subjected to a catalytic oxidative dehydrogenation in vapour phase (in 1), leading to a mixture (5) of water, of methacrylic acid and of impurities, including unconverted isobutyric acid;
   - the said mixture is condensed (in 6) and a liquid-liquid extraction is carried out (in 8) in order to separate the water (at 11) from the organic acids obtained (10);
   - an esterification (in 21) of the said acids with methanol is carried out, leading to a crude methyl methacrylate one of the impurities in which is methyl isobutyrate,

   characterised in that:
   - the liquid-liquid extraction is carried out (in 8) using a fraction (9) of the crude methyl methacrylate obtained by the esterification reaction (in 21);
   - the organic phase (10) resulting from this extraction is conveyed to a separation stage (22) situated immediately downstream of the esterification stage (21), making it possible to separate, on the one hand, the crude methyl methacrylate (23) consisting of the fraction of methyl methacrylate produced by esterification and that employed as an extraction solvent and, on the other hand, the organic acids (24) consisting of the fraction of the organic acids obtained at the end of oxidative dehydrogenation and that of the unesterified acids which are thus recycled; and
   - a purification (in 33) of the fraction of crude methyl methacrylate (32) which has not been employed as an extraction solvent is carried out in order to separate, on the one hand, pure methyl methacrylate (at 35) and, on the other hand, methyl isobutyrate (34), which is recycled to the oxidative dehydrogenation stage (1).

2. Process according to Claim 1, characterised in that the organic phase (10) originating from the liquid-liquid extraction stage (8) is freed (in 17) from the impurities consisting of acetone and methyl acetate before the said phase is sent (at 19) to the crude methyl methacrylate/organic acids separation stage (22).

3. Process according to either of Claims 1 and 2, characterised in that the crude methyl methacrylate effluent (23) is freed from its residual water (in 27) before the said effluent is sent back to the extraction stage (8) and to the crude methyl methacrylate/methyl isobutyrate separation stage (33).

4. Process according to Claim 3, characterised in that a fraction of the crude methyl methacrylate effluent, freed (in 27) from residual water, is recycled (at 30) as a reflux to the crude methyl methacrylate-

/organic acids separation stage (22).

5. Process according to one of Claims 1 to 4, characterised in that an additional purification (in 36) of the methyl methacrylate obtained at the pure methyl methacrylate/methyl isobutyrate separation stage (33) is performed, the heavy products separated (at 38) being, if appropriate, recycled to the crude methyl methacrylate/organic acids separation stage (22).

6. Process according to one of Claims 1 to 5, characterised in that the aqueous phase (11) taken from the extraction stage (in 8) is recycled to the oxidative dehydrogenation stage (1) after the said phase has been subjected to a stripping operation (in 12) making it possible to separate off an organic phase which is recycled to the esterification stage (21), and after having removed from the aqueous phase purified by the stripping operation a purge (14) which is substantially equal to the water formed by the oxidative dehydrogenation (in 1) and esterification (in 21) reactions.

7. Process according to Claims 3 and 6 taken simultaneously, characterised in that the residual water (28) extracted (in 27) from the crude methyl methacrylate effluent (23) is recycled to the stripping stage (12).

8. Process according to one of Claims 2 to 7, characterised in that the stream (20) leaving the esterification stage (19) is sent to the separation stage (22) at a point lower than that to which is sent the stream (19) originating from the liquid-liquid extraction stage (8) after having been freed (in 27) from the impurities which it contained.

9. Process according to one of Claims 2 to 7, characterised in that, before being sent to the separation stage (22), the stream (20) leaving the esterification stage (19) is combined with the stream (19) originating from the liquid-liquid extraction stage (8), after having been freed (in 27) from the impurities which it contained.

# FIG. 1

Acide isobutyrique

Air

Acétone
Acétate de méthyle

Phase aqueuse (vers 12)

Isobutyrate de méthyle recyclé

eau

vers 21

Purge d'eau

Vers purge des fractions lourdes

Méthanol frais

Méthacrylate de méthyle pur

Méthacrylate de méthyle + fractions lourdes (vers 22)

EP 0 356 315 B1